# EUROPEAN PATENT SPECIFICATION

(11) **EP 1 849 474 B1**
(45) Date of publication and mention of the grant of the patent: **07.09.2016**
(21) Application number: 06713649.9
(22) Date of filing: 14.02.2006
(51) Int. Cl.: A61K 38/00, A61K 31/7088, A61K 48/00, A61P 31/04, C07K 7/08, C12N 15/09, A61K 38/10

(54) **ANTIMICROBIAL PEPTIDE AND USE THEREOF**
ANTIMIKROBIELLES PEPTID UND VERWENDUNG DAVON
PEPTIDE ANTIMICROBIEN ET EMPLOI DUDIT PEPTIDE

(30) Priority: 15.02.2005 JP 2005037973
(43) Date of publication of application: 31.10.2007
(73) Proprietor: TOAGOSEI CO., LTD., Minato-ku Tokyo 105-8419 (JP)
(72) Inventor: YOSHIDA, Tetsuhiko, TOAGOSEI CO., LTD, Nagoya-shi, Aichi, 4550027 (JP); KOBAYASHI, Nahoko, TOAGOSEI CO., LTD, Nagoya-shi, Aichi, 4550027 (JP)
(74) Representative: Thurston, Joanna
(86) International application number: PCT/JP2006/302508
(87) International publication number: WO 2006/088010

(56) References cited:
- WO-A1-03/091429
- WO-A1-2007/010989
- WO-A2-02/02055
- WO-A2-2004/065577
- WO-A2-2005/086800
- US-A1- 2002 147 307
- SONG M M ET AL: "The suppressor of cytokine signaling (SOCS) 1 and SOCS3 but not SOCS2 proteins inhibit interferon-mediated antiviral and antiproliferative activities", JOURNAL OF BIOLOGICAL CHEMISTRY, THE AMERICAN SOCIETY OF BIOLOGICAL CHEMISTS, INC., BALTIMORE, MD, US, vol. 273, no. 52, 25 December 1998 (1998-12-25), pages 35056-35062, XP002323167, ISSN: 0021-9258, DOI: DOI:10.1074/JBC.273.52.35056
- BUTUYAN M.V. ET AL.: 'Solution Structure and Dynamics of Yeast Elongin C in Complex with a von Hippel-Lindau Peptide' JOURNAL OF MOLECULAR BIOLOGY vol. 312, 2001, pages 177 - 186, XP004466128
- KAMURA T. ET AL.: 'VHL-box and SOCS-box domains determine binding specificity for Cul2-Rbx1 and Cul5-Rbx2 modules of ubiquitin ligases' GENES & DEVELOPMENT vol. 18, no. 24, 15 December 2004, pages 3055 - 3065, XP003000935
- KAMIZONO S. ET AL.: 'The SOCS box of SOCS-1 accelerates ubiquitin-dependent proteolysis of TEL-JAK2' JOURNAL OF BIOLOGICAL CHEMISTRY vol. 276, no. 16, 2001, pages 12530 - 12538, XP002240718
- BUCHBERGER ALEXANDER ET AL: "Biophysical characterization of elongin C from Saccharomyces cerevisiae", BIOCHEMISTRY, vol. 39, no. 36, 12 September 2000 (2000-09-12), pages 11137-11146, ISSN: 0006-2960

## Description

### [Field of the Invention]

The present invention relates to oligopeptides or polypeptides that are composed of an independent peptide chain not present in nature and that have antimicrobial property (hereinafter, collectively referred to as an "antimicrobial peptide"), and their utilization. More specifically, the present invention relates to antimicrobial agents (compounds) having such an antimicrobial peptide as the main component.

This present application claims priority based on Japanese Patent Application No. 2005-037973 filed on February 15, 2005, the entire content of which is hereby incorporated by reference.

### [Background of the Invention]

It is generally considered that antimicrobial peptides have a broad antimicrobial spectrum and that drug-resistant bacterial strains rarely appear to arise, and therefore antimicrobial peptides are expected to be used for the purpose of preventing and treating bacterial infectious diseases in humans and animals or providing antimicrobial properties to products such as food. A large number of antimicrobial peptides have been isolated from various animals and plants to date. For example, a variety of naturally derived antimicrobial peptides are disclosed in Japanese Patent Applications Nos. 2000-63400 and 2001-186887, and International Publications Nos. WO98/51794, WO99/26971, WO00/09553, WO00/59527 and WO01/09175.

In addition, antimicrobial peptides designed and synthesized utilizing known amino acid sequences of which the relationships to their antimicrobial properties have never been discussed are reported in International Publication No. WO03/91429.
US 2002/147307 describes therapeutic and diagnostic agents comprising a SOCS box. These may be used to modulate a wide range of cellular responses. Buchberger A et al (Biochemistry (2000) 39, 11137-11146) describes a VHL peptide corresponding to residues 157-171 of human VHL.

### [Summary of the Invention]

An object of the present invention is to design new antimicrobial peptides different from peptides existing and functioning as antimicrobial peptides in nature, not by conventional approaches as described in the above publications to develop antimicrobial agents containing antimicrobial peptides. Another object of the present invention is to produce antimicrobial peptides designed by the present invention and to provide antimicrobial agents (pharmaceutical compositions) containing the peptide as a main component. Another object of the present invention is to provide polynucleotides encoding the antimicrobial peptides described herein.

The antimicrobial peptides provided by the present invention are artificial antimicrobial peptides designed by utilizing the amino acid sequences contained in polypeptides that are different from polypeptides present as antimicrobial peptides in nature.

The present inventors focused on a region of von Hippel Lindau (VHL) protein, which is a product of VHL gene, which is a gene involved in VHL disease and is known as an antioncogene, the region being a region (i.e., α-domain) which may be coupled to Elongin BC complex (specifically, part of Elongin C) known to form a complex with Elongin A and act as a transcriptional regulator. See, for example, C. E. Stebbins, W.G. Kaelin Jr., and N. P. Pavletich, SCIENCE, Vol. 284, 1999, pp. 455-461, and M. Ohh, Y. Takagi, T. Aso, C. E. Stebbins, N. P. Pavletich, B. Zbar, R. C. Conaway, J. W. Conaway, and W. G. Kaelin Jr., The Journal of Clinical Investigation, Vol. 104, 1999, pp. 1583-1591.

The present inventors further focused on a region ("SOCS-box") considered to coupled with Elongin BC, the region being of SOCS (suppressor of cytokine signaling) protein and its family members which contain a structural motif (i.e., SOCS-box) similar to the above region (the VHL region) See, for example, the above document by Stebbins et al., and the documents T. Kamura, S. Sato, D. Haque, L. Liu, W. G. Kaelin Jr., R. C. Conaway, and J. W. Conaway, GENES & DEVELOPMENT, Vol. 12, 1998, pp. 3872-3881, and D. J. Hilton, R. T. Richardson, W. S. Alexander, E. M. Viney, T. A. Willson, N. S. Sprigg, R. Starr, S. E. Nicholson, D. Metcalf, and N. A. Nicola, PNAS, Vol. 95, 1998, pp. 114-119.

The present inventors found that peptides including a partial sequence of the amino acid sequence in these regions (domain or motif) exhibit high antimicrobial activity against microorganisms such as bacteria, thereby leading to the present invention.

Antimicrobial peptides described herein are artificially synthesized antimicrobial peptides that are not present in nature and have antimicrobial property against at least one kind of bacteria.

Several antimicrobial peptides described herein are peptides having, as the amino acid sequence related to the expression of antimicrobial property, the amino acid sequence constituting the α-domain of human-derived VHL protein (hereinafter simply referred to as "VHL") and being included in the region involved in coupling of VHL and Elongin C, or a partially-modified sequence of such amino acid sequence (for example, a sequence in which one to several amino acid residues are substituted in, deleted from and/or added to the sequence). In addition, several antimicrobial peptides disclosed herein are peptides having, as the amino acid sequences related to the expression of antimicrobial property, a partial amino acid sequence of the amino acid sequence constituting SOCS-box (which is composed of approximately 40 to 50 amino acid residues) of several proteins belonging to the group of proteins having the region called as SOCS-box (including the BC-box motif), which hereinafter are collectively referred as "SOCS-box protein," or an amino acid sequence partially modified from such sequence (for example, a sequence in which one to several amino acid residues are substituted in, deleted from and/or added to the sequence).

Described herein is a pharmaceutical composition for use in preventing or treating a bacterial infection, the composition comprising a peptide having antimicrobial property against at least one kind of bacteria and a pharmaceutically acceptable carrier, wherein the peptide is composed of an amino acid sequence selected from the amino acid sequences below :-
**(a)** TLKERCLQVVRSLVK (SEQ ID NO: **1**);
**(b)** SLQYLCRFVIRQYTR (SEQ ID NO: **2**);
**(c)** SLQHICRMSIRRVMS (SEQ ID NO: **3**):
**(d)** TLLSLCRVAVRRALG (SEQ ID NO: **4**);
**(e)** TLKKRCLQVVRKLVK (SEQ ID NO: **5**);
**(f)** TLQHLCRKTVNGHLD (SEQ ID NO: **7**):
**(g)** SLQHICRTVICNCTT (SEQ ID NO: **8**);
**(h)** SLQYICRAVICRCTT (SEQ ID NO: **9**);
**(i)** SLQHLCRFRIRQLVR (SEQ ID NO: **10**);
**(j)** SLKHLCRKALRSFLT (SEQ ID NO: **11**);
**(k)** PLAHLCRLRVRKAIG (SEQ ID NO: **12**); and
**(l)** SLTHLCRLEIRSSIK (SEQ ID NO: **13**),
or a modified amino acid sequence of the selected amino acid sequence in which one, two or three amino acid residues are conservatively substituted.

In this specification, "artificially synthesized antimicrobial peptides that are not present in nature" refers to peptide moieties whose peptide chain alone does not independently or stably exist in nature, but is artificially produced by chemical synthesis or biosynthesis (i.e., production based on genetic engineering) and can exist stably in a prescribed system (for example, a composition constituting an antimicrobial agent).

In this specification, "antimicrobial peptide" is a term referring to an amino acid polymer having a plurality of peptide bonds displaying antimicrobial activity against at least one kind of microbe, and is not limited by the number of amino acid residues constituting the peptide chain. Also described herein are antimicrobial peptides in this specification including oligopeptides having ten or less amino acid residue(s) or polypeptides containing more than ten amino acid residues.

In this specification, "amino acid residue" is a term encompassing the N-terminal amino acid and C-terminal amino acid of the peptide chain, unless otherwise specified.

In this specification, with respect to a specific amino acid sequence, "a sequence partially modified (modified amino acid sequence)" refers to an amino acid sequence formed by substituting, deleting or adding (inserting) one, two or three acid residue(s) without losing the antimicrobial property of the specific amino acid sequences. For example, a sequence formed by the so-called conservative amino acid replacement in which one to three amino acid residues are conservatively substituted (for example, a sequence in which a basic amino acid residue is substituted with another basic amino acid residue); a sequence formed by adding to or removing from the specific sequence one, two or three amino acid residue(s); or a sequence in which a non-basic amino acid residue (for example, serine, threonine, aspartic acid, glutamic acid) is substituted with a basic amino acid residue (lysine, arginine); or the like is a typical example included in the "sequence being partially modified (modified amino acid sequence)" of this specification.

The present inventors identified amino acid sequences that are present in the region (motif) considered to couple with Elongin BC complex (specifically, part of the amino acid sequence in the Elongin C portion) and that may serve as the amino acid sequence capable of exhibiting antimicrobial property in a relatively short peptide chain that can be artificially synthesized. The amino acid sequences shown in the above **(a)** to **(d)** and **(f)** to **(1)** are typical examples. Specifically, the amino acid sequence (15 amino acid residues) shown in **(a)** is a sequence contained in the α-domain of human VHL. The sequences shown in **(b), (c), (d), (f), (g), (h), (i), (j), (k)** and **(l)** are the sequences contained in the SOCS-box of hSOCS-6 (b), , mWSB-1 **(c),** mASB-1 **(d),** mSOCS-3 **(f),** mSOCS-4 **(g),** mSOCS-5 **(h),** mSOCS-7 **(i),** mWSB-2 **(j),** mASB-2 **(k)** and hASB-3 **(1),** respectively, which are identified each as a SOCS-box protein (see above **3** and **4**). The amino acid sequence shown in the above **(e)** is a modified sequence based on the amino acid sequence shown in the above **(a)** (i.e., a modified sequence in which the glutamic acid residue and the serine residue, which are the fourth and the twelfth amino acid residues, respectively from the N-terminal of the amino acid sequence shown in **(a),** are each substituted with a lysine residue).

Also described herein are antimicrobial peptides of which at least one of the amino acid residues is amidated. Amidating carboxyl group of amino acid residues (typically a C-terminal amino acid residue of the peptide chain) can improve the structural stability (for example, protease resistance).

Also described herein are antimicrobial peptides of which the total number of amino acid residues constituting the peptide chain is 100 or less (or 50 or less, or 30 or less, or 20 or less).Such short-chained peptides are easily chemically synthesized, and can easily provide antimicrobial peptides.

Also described herein are antimicrobial agents (typically a compound that may be utilized in the medical or sanitary field) containing, as the main component, an antimicrobial peptide that has antimicrobial property against at least one kind of bacteria, and the antimicrobial peptide being one, two or more kinds of the antimicrobial peptides described herein. The antimicrobial agent contains an artificially synthesized antimicrobial peptide (preferably, the total number of amino acid residues constituting the peptide chain is 30 or less) having either one of the amino acid sequences represented by the above (a), (b), (c), (d), (f), (g), (h), (i), (j), (k) and (l); or such a sequence with partial modification (for example, the amino acid sequence represented by the above (e)).

Antimicrobial agents provided by the present invention may exhibit high antimicrobial activity against at least one type of bacteria (Gram-positive bacteria and/or Gram-negative bacteria), or fungi.

Also described herein are antimicrobial agents which include one or more kinds of antimicrobial peptides and a carrier that may be pharmaceutically acceptable.

Also described herein are antimicrobial peptides (of which the total amino acid residues constituting the peptide chain is 30 or less) having an amino acid sequence represented by either one of SEQ ID NOs 1, 2, 3, 4, 5, 7, 8, 9, 10, 11, 12 and 13 (therefore, amino acid sequences represented by the above (a) to (l)), or an amino acid sequence formed by substituting in, deleting from or adding to such amino acid sequence one or several amino acid residues. As an example, may be given a peptide being constituted of 30 or less (preferably 20 or less) amino acid residues in total and containing an amino acid sequence represented by SEQ ID NO 5 (the amino acid sequence represented by (e)) or an amino acid sequence formed by substituting in, deleting from or adding to the amino acid sequence one or several amino acid residues (though sequences equivalent to the amino acid sequence represented by SEQ ID NO 1 are excluded). Antimicrobial peptides containing such sequence (typically peptides composed of such sequence) can exhibit high antimicrobial activity against bacteria, particularly Gram-positive bacteria such as Staphylococcus aureus. Therefore, antimicrobial agents containing such peptide(s) are favorable.

Further, the present invention provides a method for producing the antimicrobial peptides described herein. Particularly, the method for producing antimicrobial peptide provided by the present invention includes designing a peptide chain having antimicrobial property against at least one kind of bacteria, the method comprising designing a peptide composed of the amino acid sequence TLKKRCLQVVRKLVK (SEQ ID NO: 5) and synthesizing the designed peptide chain.

Also described here are methods for producing antimicrobial peptides provided by the present invention which includes designing a peptide chain having antimicrobial property against at least one kind of bacteria and having either one of the amino acid sequences represented by the above (a), (b), (c), (d), (f), (g), (h), (i), (j), (k) and (l) or such sequences with partial modification (for example, an amino acid sequence represented by (e) or an amino acid sequence formed by substituting in, deleting from or adding to such amino acid sequence one or several amino acid residues); and synthesizing the designed peptide chains.

In designing such peptide chain, the peptide chain is designed in such that the total number of amino acid residues constituting the peptide chain is 30 or less (or 20 or less).

The present invention also provides artificially designed polynucleotides that are not present in nature; and consist of a nucleotide sequence encoding the peptide sequence TLKKRCLQVVRKLVK (SEQ ID NO: 5) and/or a nucleotide sequence complementary to said nucleotide sequence (for example, the designed polynucleotide being a polynucleotide substantially composed of such sequence).

In this specification, "polynucleotide" is a general term referring to a polymer (nucleic acid) in which a plurality of nucleotides are coupled via phosphodiester bond, and is not limited by the number of the nucleotides. The polynucleotides in this Specification include DNA and RNA fragments of various lengths. In addition, "artificially designed antimicrobial polynucleotide that does not occur naturally" refers to an antimicrobial polynucleotide whose nucleotide chain (the total length) alone is not present in nature, but is artificially produced by chemical synthesis or biosynthesis (i.e., production based on genetic engineering).

There is no specific limitation in selecting a codon that defines each amino acid, and selection may be made with consideration of the frequency of usage in the applicable host cells.

**<Sequence Listing Free Text>**

| | |
|---|---|
| SEQ ID NO:1 | designed antimicrobial peptide containing an amide group in its terminal |
| SEQ ID NO:2 | designed antimicrobial peptide containing an amide group in its terminal |
| SEQ ID NO:3 | designed antimicrobial peptide containing an amide group in its terminal |
| SEQ ID NO:4 | designed antimicrobial peptide containing an amide group in its terminal |
| SEQ ID NO:5 | designed antimicrobial peptide containing an amide group in its terminal |
| SEQ ID NO:6 | designed peptide containing an amide group in its terminal |
| SEQ ID NO:7 | designed antimicrobial peptide containing an amide group in its terminal |
| SEQ ID NO:8 | designed antimicrobial peptide containing an amide group in its terminal |
| SEQ ID NO:9 | designed antimicrobial peptide containing an amide group in its terminal |
| SEQ ID NO:10 | designed antimicrobial peptide containing an amide group in its terminal |
| SEQ ID NO:11 | designed antimicrobial peptide containing an amide group in its terminal |
| SEQ ID NO:12 | designed antimicrobial peptide containing an amide group in its terminal |
| SEQ ID NO:13 | designed antimicrobial peptide containing an amide group in its terminal |

### [Best Mode for Carrying Out the Invention]

Hereinafter, preferred embodiments of the present invention will be described. The matters that are apart from those specifically mentioned in this specification (for example., primary structure or chain length of an antimicrobial peptide) and are necessary for performing the present invention (for example, general matters related to peptide synthesis, polynucleotide synthesis, and preparation of antimicrobial agent (pharmaceutical compound) including peptide as a component) can be considered as matters of design by those skilled in the art based on the conventional technology in the fields of organic chemistry, biochemistry, genetic engineering, protein engineering, molecular biology, pharmaceuticals, medicine, hygiene and the like. The present invention can be performed based on the contents described in this specification and the common technical knowledge in the field. In the following description, where appropriate, amino acids are also expressed in one-letter codes based on the IUPAC-IUB nomenclature for amino acids (three-letter codes in Sequence List).

The antimicrobial peptides described in the present specification are artificially designed peptides that are not present in nature, and relatively short polypeptides or oligopeptides having, as the amino acid sequence related to the expression of its antimicrobial property, either one of the amino acid sequences represented by the above (a), (b), (c), (d), (f), (g), (h), (i), (j), (k) and (l); or sequences such as the amino acid sequence represented by the above (e), that are resulting from partial modification of the above amino acid sequences (hereinafter, the amino acid sequences related to the present invention will be collectively referred to as "antimicrobial associated sequences"). Preferred are peptides of which 50% or greater of the total amino acid residues constituting the peptide chain is composed of one, two or more units of the antimicrobial associated sequences. Here, one unit of antimicrobial associated sequence refers to one sequence portion (region or motif) constituting the antimicrobial associated sequence. Therefore, when a peptide chain contains two units of antimicrobial associated sequences, it means that in the peptide chain, regardless of whether the same or different, there are present two sequences that can be mutually and independently considered as antimicrobial associated sequences, . Peptides having short peptide chain composed of one unit of antimicrobial associated sequence are typical examples of the antimicrobial peptides described herein, and such antimicrobial peptides are preferable to be utilized as main component of antimicrobial agents (see Examples described below).

There is no specific limitation to the proportion of the antimicrobial associated sequence in the entire amino acid sequence (i.e. of the total number of amino acid residues constituting the peptide chain, the percentage that is accounted for by the number of amino acid residues constituting the antimicrobial associated sequence) if 50% or greater, but more preferable is 70% orgreater, or particularly preferable is 90% or greater. As for the antimicrobial peptides described by the present invention, it is preferable for all the amino acid residues to be L-amino acids, but a part or all of the amino acid residues may be substituted with D-amino acids as long as the antimicrobial activity is not lost.

The chain length (thus the total number of amino acid residues) of the antimicrobial peptide described herein can vary according to the length of the antimicrobial associated sequence, and therefore it is not particularly limited to, but it is appropriate to be 100 or less (typically 50 or less), and is preferred to be 30 or less (particularly preferred to be 20 or less). For example, an antimicrobial peptide composed of 10 to 20 amino acid residues displays high antimicrobial activity, and at the same time is easy to synthesize and utilize. There is no particular limitation regarding the conformation (three-dimensional structure) of the peptide, as long as the peptide exhibits antimicrobial activity in the environment in which it is used, but in terms that it hardly becomes an immunogen (antigen), it is preferred to be either linear or helical. Peptides in these conformations hardly form epitopes. In this view, it is desirable for an antimicrobial peptide applied to antimicrobial agent to be linear and has a relatively low molecular weight (typically, the number of amino acid residues is 30 or less (particularly 20 or less, for example, 14, 15, 16 or 17)).

As for the antimicrobial associated sequence for designing antimicrobial peptides of this invention, any amino acid sequence selected from the above-described (a), (b), (c), (d), (f), (g), (h), (i), (j), (k) and (l) can be utilized. Alternatively, antimicrobial peptides (peptide chains) can be designed utilizing a modified sequence , for example, a sequence resulted from substituting, deleting or adding one or a plurality (typically nine or less, preferably about two to five) of amino acid residues. For example, any amino acid sequence selected from the above-described (a), (b), (c), (d), (f), (g), (h), (i), (j), (k) and (l) can be utilized as a basis for creating a modified sequence, and the sequence can be modified using antimicrobial activity as an index (for example, via MIC testing against bacteria). Specific methods for modification include substitution, deletion or addition of amino acid residue(s). In other words, basing on any amino acid sequence selected from the above-described (a), (b), (c), (d), (f), (g), (h), (i), (j), (k) and (l) (or the amino acid sequence of (e)), substitution, deletion or addition (insertion) of one or a plurality of amino acid residue(s) is optionally carried out, and are produced peptides composed of such modified sequence(s), on which a prescribed antimicrobial activity test is run (see Examples described below). For example, one amino acid sequence from the above-described (a), (b), (c), (d), (f), (g), (h), (i), (j), (k) and (l), which are composed of 15 amino acid residues, is first selected (for example, sequence (a) is selected), and many modified sequences can be designed by substituting a few amino acid residues in the selected amino acid sequence (for example, the N-terminal amino acid residue, threonine, of the selected sequence (a)) with amino acid residues located at the corresponding positions in another sequence (for example, the N-terminal amino acid residue, serine, of the sequence (b)). Thereafter, peptides composed of the obtained modified sequence are produced and on these is run a prescribed antimicrobial activity test. Accordingly, can be determined if it possesses antimicrobial activity or not.

Alternatively, in terms to reduce production cost or to facilitate chemical synthesis, deletion of amino acid residue(s) is preferable. In terms to increase the structural stability, addition of amino acid residue(s) is preferable. In terms to improve antimicrobial activity, substitution of amino acid residue(s) is preferable. Particularly preferable modified sequences include, for example, sequences of which one or a plurality of non-basic amino acid residue(s) is substituted with basic amino acid residue(s) (for example, the amino acid sequence in the above (e)).

The antimicrobial peptides described herein can partially contain sequences that are not included in the antimicrobial associated sequences as long as antimicrobial property is not lost. Although there is no specific limitation, for such partial sequences, sequences that can allow to maintain the three-dimensional conformation (typically the linear chain form) of the antimicrobial associated sequence portion in the peptide chain.

Among the antimicrobial peptides described herein, those with relatively short peptide chain can be easily produced according to general chemical synthesis methods. For example, either a conventionally known solid-phase synthesis or liquid-phase synthesis can be employed. Solid-phase synthesis using either Boc (t-butyloxycarbonyl) or Fmoc (9-fluorenylmethoxycarbonyl) as the protecting group for amino group is appropriate.

For the antimicrobial peptides described herein, peptide chains having a desired amino acid sequence and a modified (for example, C-terminal amidated) portion can be easily synthesized by solid-phase synthesis using a commercially available peptide synthesizer (for example, available from PerSeptive Biosystems, Applied Biosystems or the like).

Alternatively, the antimicrobial peptides can be synthesized biologically based on genetic engineering procedure. This approach is preferable in producing polypeptides having a relatively long peptide chain. In other words, DNA of a nucleotide sequence (including ATG start codon) that encodes the amino acid sequence of a desired antimicrobial peptide is synthesized. A recombinant vector having gene constructs for expression including this DNA and various regulatory elements (including promoter, ribosome binding site, terminator, enhancer, various cis-elements for controlling the expression level) is constructed in accordance with the host cells.

This recombinant vector is transduced into the host cells (for example, yeast, insect cell, plant cell, animal (mammalian) cell) by a general technique, and tissues or organisms containing the host cells or the cells are cultured under predetermined conditions. Thus, the desired polypeptide can be expressed and produced in the cells. By isolating and purifying the polypeptide from the host cells (from the culture medium when secreted), can be obtained the desired antimicrobial peptide

Regarding the methods for constructing recombinant vector and methods for transducing the constructed recombinant vector into the host cell, conventional methods in this field can be utilized, and since such methods are not specific features of the present invention, detail descriptions will be omitted.

For example, a fusion protein expression system can be utilized for efficient mass production in host cells. In other words, a gene (DNA) encoding the amino acid sequence of a desired antimicrobial peptide is chemically synthesized, and the synthesized gene is cloned into a preferred site of a suitable vector for expression of a fusion protein (for example, a vector for expression of GST (Glutathione S-transferase) fusion protein such as pET series provided by Novagen and pGEX series provided by Amersham Bioscience). Then, the host cell (typically E. coli) is transformed with the vector. The obtained transformant is cultured so that the desired fusion protein is prepared. Then, the protein is extracted and purified. Then, the obtained purified fusion protein is digested with a predetermined enzyme (protease) and the separated desired peptide fragment (designed antimicrobial peptide) is recovered by affinity chromatography or the like. Antimicrobial peptides of this present invention can be produced using such conventionally known system for expression of a fusion protein (for example, GST/His system provided by Amersham Bioscience can be utilized).

Alternatively, a template DNA (i.e., a synthesized gene fragment containing a nucleotide sequence encoding the amino acid sequence of an antimicrobial peptide) for cell-free protein synthesis system is constructed, and with various compounds (ATP, RNA polymerase, amino acids and the like) necessary for peptide synthesis, a target polypeptide can be synthesized in vitro by using a so-called cell-free protein synthesis system. Regarding the cell-free protein synthesis system, for example, an article by Shimizu et al. (Shimizu et al., Nature Biotechnology, 19, 751-755 (2001)), and an article by Madin et al. (Madin et al., Proc. Natl. Acad. Sci. USA, 97(2), 559-564 (2000)) can be used as references. At the time of filing the present application, a large number of companies provide contract manufacturing service of polypeptides based on the technologies described in these articles, and kits for cell-free protein synthesis (for example, PROTEIOS (trademark) Wheat germ cell-free protein synthesis kit available from TOYOBO Co., Ltd. in Japan) are commercially available.

Therefore, once an amino acid sequence to be utilized is decided and a peptide chain is designed as described above, then a desired antimicrobial peptide can be easily produced by cell-free protein synthesis system according to the amino acid sequence. For example, the antimicrobial peptide can be easily produced based on PURESYSTEM (registered trademark) of POST GENOME INSTITUTE CO. LTD. in Japan.

A single- or double stranded polynucleotide containing a nucleotide sequence encoding an antimicrobial peptide described herein and/or a nucleotide sequence complementary to said sequence can be produced (synthesized) by a conventionally known method. In other words, a nucleotide sequence corresponding to the amino acid sequence of the antimicrobial peptide can be easily determined and provided by selecting a codon for each amino acid residue constituting the designed amino acid sequence. Then, once the nucleotide sequence is determined, then a polynucleotide (single strand) corresponding to the desired nucleotide sequence can be easily obtained utilizing a DNA synthesizer or the like. Furthermore, using the obtained single-stranded DNA as a template, the target double-stranded DNA can be obtained by using various methods for enzymatic synthesis (typically PCR).

The polynucleotides provided by the present invention may be in the form of DNA or RNA (mRNA or the like). The DNA can be provided as a double or a single strand. When it is provided as a single strand, it may be a coding chain (sense chain) or its complementary non-coding chain (anti-sense chain).

The polynucleotides provided by the present invention, as described above, can be used as a material for constructing a recombinant gene (expression cassette) for producing an antimicrobial peptide in various host cells or cell-free protein synthesis systems.

According to the present invention, are provided polynucleotides containing a nucleotide sequence encoding an antimicrobial peptide of a novel amino acid sequence (for example, an antimicrobial peptide composed of such as the amino acid sequence represented by (e), a modified sequence of each amino acid sequence represented by the above-described (a), (b), (c), (d), (f), (g), (h), (i), (j), (k) or (l)), and/or a nucleotide sequence complementary to said nucleotide sequence. For example, is provided an artificially designed polynucleotide that is not present in nature, the polypeptide of which the total number of amino acid residues constituting the peptide chain is 50 or less (preferably 30 or less, and particularly preferably 20 or less) containing a nucleotide sequence encoding an amino acid sequence, which includes an amino acid sequence represented by either one of SEQ ID NOs 1, 2, 3, 4, 5, 7, 8, 9, 10, 11, 12 and 13 or a modified sequence (antimicrobial associated sequence) of such sequence and/or a nucleotide sequence complementary to said nucleotide sequence (or substantially composed of such sequence).

Antimicrobial peptides described herein possess high antimicrobial activity against at least one kind of microbe; and those preferred have a relatively broad antimicrobial spectrum and can be used preferably as the main component of antimicrobial agent. For example, it can be used for the purpose of treating bacterial infections, disinfecting wounds, preventing eye diseases, cleaning the oral cavity (gargling), preventing deterioration of food, preserving freshness of food, removing odors, desinfecting the surface of furniture or sanitary equipment, and the like.

The antimicrobial peptide to be contained in antimicrobial agent may be in the form of a salt as long as the antimicrobial property is not inhibited. For example, can be used an acid salt of the peptide obtained by carrying out addition reaction with a generally used non-organic or organic acid following a conventional procedure. Alternatively, it can be other kinds of salts (for example metal salts) as long as its antimicrobial activity is retained.

Antimicrobial agents used for such purposes may include, apart from an antimicrobial peptide as the main component, various carriers that can be medically (pharmaceutically) acceptable. Carriers that are generally used as diluent, excipient, or the like in peptide pharmaceuticals are desirable. Although it may vary according to the application or form of the antimicrobial agent, typically, water, physiological buffer or various organic solvents are used. For example, an aqueous solution of an alcohol (such as ethanol) at an appropriate concentration, glycerol, a non-drying oil such as olive oil may be used. Alternatively, liposome may also be used. Sub-components that may be contained in the antimicrobial agent include, various filling agents, fillers, binders, moisturizers, surfactants, colors, fragrances or the like.

There is no particular limitation regarding the form of the antimicrobial agent. For example, typical pharmaceutical formulations for internal or external use are an ointment, liquid, suspension, emulsion, aerosol, foam, granule, powder, tablet, and capsule. Furthermore, to be used for injections and the like, it can be formulated as a freeze dried or granulated product that is to be dissolved in a physiological saline or an appropriate buffer (for example PBS) or the like immediately before use so as to prepare a medical fluid. Carriers to be contained in an antimicrobial agent may vary according to the form of the antimicrobial agent.

The process to prepare various forms of pharmaceuticals from the antimicrobial peptide (main component) and various carriers (secondary component) as ingredients can be carried out according to a conventionally known method, and because such process itself does not characterize the present invention, its detailed description will be omitted. As a detailed information source related to formulations, for example, can be referred to "Comprehensive Medicinal Chemistry" edited by Corwin Hansch and published by Pergamon Press (1990).

Antimicrobial agents provided by the present invention can be used in a way or a dose in accordance with their forms and purposes.

Antimicrobial peptides containing an antimicrobial associated sequence described herein can maintain high antimicrobial activity even in a system where a relatively high concentration of cation, saline (for example sodium chloride) or organic compound such as serum is present. Hence it is also preferable for the antimicrobial peptide described herein to be used in systems (locations) where cation, saline, serum or the like is present. For example, antimicrobial agents provided by the present invention can be, as a liquid agent, administered to a patient, by intravenous, intramuscular, subcutaneous, intracutaneous or intraperitoneal injection, or by enema.

Alternatively, those in a solid form such as tablet can be orally administered. For use in sterilizing (disinfecting) the surface of sanitary ceramic ware or preventing deterioration of food, a liquid agent containing a relatively large amount (for example, 1 to 100 mg/ml) of antimicrobial peptide can be sprayed directly onto the surface of a target object, or the surface of the target object can be wiped with fabric or paper impregnated with the liquid agent. These are only examples, so that the formulation and usage same as those of conventional peptide antibiotics; or pesticides, quasi-drugs or the like composed of peptides can be applied.

For example, for cancer or aids patients in radiotherapy, prevention and treatment of bacterial infections are of great interest. The antimicrobial peptides described herein can exhibit high antimicrobial effect against bacteria responsible for infectious diseases (for example, Gram-positive bacteria such as Staphylococcus aureus). Therefore, the antimicrobial peptides of the present invention are useful as a main component of antimicrobial agent.

Polynucleotides encoding the antimicrobial peptides of the present invention can be used as a material for so-called gene therapy. For example, a gene (typically DNA segment, or RNA segment) encoding an antimicrobial peptide is cloned into a suitable vector, and transduced into a target part, so that the antimicrobial peptide related to the present invention can be expressed constantly in an organism (cells). Therefore, a polynucleotide (a DNA segment, RNA segment or the like) encoding an antimicrobial peptide of the present invention is useful as a pharmaceutical for preventing or treating bacterial infections in the above-described patients or the like.

In the field of regenerative medicine, it is important to prevent bacterial infections during cultivation of skin, bone or various organs. The antimicrobial peptides described herein are extremely low in toxicity against mammalian cells or tissues and can exhibit antimicrobial effects selective to bacteria. Therefore, they are very useful as pharmaceuticals to prevent bacterial infections in cultured organs or the like. For example, as shown in the examples described later, bacterial infections in an organ being cultured can be prevented by adding at an appropriate concentration to the culture medium an antimicrobial peptide of the present invention by itself or an antimicrobial agent containing the peptide as one of the main components.

Furthermore, with respect to cultured cells or cultured tissues, a polynucleotide encoding an antimicrobial peptide of the present invention can be used as a material for gene therapy. For example, a gene (typically a DNA segment, or RNA segment) encoding an antimicrobial peptide of the present invention is cloned into a suitable vector, and transduced into a target cultured tissue, so that the antimicrobial peptide related to the present invention can be expressed constantly or during a desired time period in the cultured tissue (or cells). Therefore, a polynucleotide (a DNA segment, RNA segment or the like) encoding an antimicrobial peptide of the present invention is useful as a pharmaceutical to prevent or treat bacterial infections in cultured tissues.

Hereinafter, several examples related to the present invention will be described, but they are not intended to limit the present invention.

### < Example 1: Synthesis of peptides>

6 kinds of peptides (samples 1 to 5, comparative sample 1) were produced using a peptide synthesizer described later. Table 1 shows the amino acid sequences of these synthetic peptides.

[Table 1]

**Table 1**

| Sample No. | Amino Acid Sequence | Total Number of Amino Acid Residues |
|---|---|---|
| sample 1 | TLKERCLQVVRSLVK (SEQ ID NO: 1) | 15 |
| sample 2 | SLQYLCRFVIRQYTR (SEQ ID NO: 2) | 15 |
| sample 3 | SLQHICRMSIRRVMS (SEQ ID NO: 3) | 15 |
| sample 4 | TLLSLCRVAVRRALG (SEQ ID NO: 4) | 15 |
| sample 5 | TLKKRCLQVVRKLVK (SEQ ID NO: 5) | 15 |
| sample 6 | TLQHLCRKTVNGHLD (SEQ ID NO: 7) | 15 |
| sample 7 | SLQHICRTVICNCTT (SEQ ID NO: 8) | 15 |
| sample 8 | SLQYICRAVICRCTT (SEQ ID NO: 9) | 15 |
| sample 9 | SLQHLCRFRIRQLVR (SEQ ID NO: 10) | 15 |
| sample 10 | SLKHLCRKALRSFLT (SEQ ID NO: 11) | 15 |
| sample 11 | PLAHLCRLRVRKAIG (SEQ ID NO: 12) | 15 |
| sample 12 | SLTHLCRLEIRSSIK (SEQ ID NO: 13) | 15 |
| | | |
| comparative sample 1 | TLHQQCIRVLKNNID (SEQ ID NO: 6) | 15 |

As shown in Table 1, every sample from 1 through 12 includes an antimicrobial associated sequence. Particularly, the peptide in sample 1 (SEQ ID NO. 1) is a peptide composed of 15 amino acid residues arranged in the amino acid sequence represented by (a). The peptide in sample 2 (SEQ ID NO. 2) is a peptide composed of 15 amino acid residues arranged in the amino acid sequence represented by (b). The peptide in sample 3 (SEQ ID NO. 3) is a peptide composed of 15 amino acid residues arranged in the amino acid sequence represented by (c). The peptide in sample 4 (SEQ ID NO. 4) is a peptide composed of 15 amino acid residues arranged in the amino acid sequence represented by (d). The peptide in sample 5 (SEQ ID NO. 5) is a peptide composed of 15 amino acid residues arranged in the amino acid sequence represented by (e). The peptide in sample 6 (SEQ ID NO. 7) is a peptide composed of 15 amino acid residues arranged in the amino acid sequence represented by (f). The peptide in sample 7 (SEQ ID NO. 8) is a peptide composed of 15 amino acid residues arranged in the amino acid sequence represented by (g). The peptide in sample 8 (SEQ ID NO. 9) is a peptide composed of 15 amino acid residues arranged in the amino acid sequence represented by (h). The peptide in sample 9 (SEQ ID NO. 10) is a peptide composed of 15 amino acid residues arranged in the amino acid sequence represented by (i). The peptide in sample 10 (SEQ ID NO. 11) is a peptide composed of 15 amino acid residues arranged in the amino acid sequence represented by (j). The peptide in sample 11 (SEQ ID NO. 12) is a peptide composed of 15 amino acid residues arranged in the amino acid sequence represented by (k). The peptide in sample 12 (SEQ ID NO. 13) is a peptide composed of 15 amino acid residues arranged in the amino acid sequence represented by (l).

On the other hand, the peptide in comparative sample 1 (TLHQQCIRVLKNNID: SEQ ID NO. 6) is a synthetic peptide composed of a partial sequence of human Elongin A (see the above document of Kamura et al.). Note that, in every sample, the carboxyl group (-COOH) of the C-terminal amino acid is amidated (-CONH₂).

Each of the above-described peptide was synthesized by solid phase synthesis (Fmoc chemistry) using a commercially available peptide synthesizer (PEPTIDE SYNTHESIZER 9050 manufactured by PerSeptive Biosystems). As the coupling agent, HATU (available from Applied Biosystems) was used, and the resin and the amino acids used in the solid phase synthesis were purchased from NOVA biochem. To amidate the C-terminal of the amino acid sequence, "Rink Amide resin (100 to 200 mesh)" was used as the solid phase carrier.

The peptide chain was extended from the Fmoc-amino acid coupled to the resin by repeating deprotection and coupling reactions according to the synthesis program of the above-described peptide synthesizer, so that the synthetic peptide of a target length was obtained. More specifically, Fmoc as an amino protecting group of amino acid, was cleaved with 20% piperidine/dimethylformamide (DMF) (peptide synthesis grade manufactured by KANTO KAGAKU), washed with DMF, reacted with 4eq of Fmoc-amino acid (-OH) each, and washed with DMF. This operation was repeated. Then, after all the extension reaction of the peptide chain was completed, the Fmoc group was cleaved with 20% piperidine/DMF, and the above-described product was washed with DMF and methanol in this order.

After the solid phase synthesis, the synthetic peptide chain on the resin was transferred to a centrifuge tube, and 1.8 mL of ethanediol, 0.6 mL of m-cresol, 3.6 mL of thioanisole, and 24 mL of trifluoroacetic acid were added thereto, and then the mixture was stirred at room temperature for two hours. Thereafter, the resin that had been coupled to the peptide chain was removed by filtration.

Then, cooled ethanol was added to the filtrate, and cooled with ice-cold water, a peptide precipitate was obtained. Thereafter, the supernatant was discarded after centrifugation (at 2500 rpm for five minutes). Cooled diethyl ether was added to the precipitate and stirred sufficiently, and then centrifugation was carried out in the same conditions as above. This process of stirring and centrifugation was repeated three times in total.

The obtained peptide precipitate was vacuum-dried and purified by high performance liquid chromatography (Waters 600 manufactured by Waters Corp.).

More specifically, a pre-column (Guard-Pak Delta-pak C18 A300 manufactured by Nippon Waters) and a C18 reverse phase column (XTerra (trademark) column, MS C18, 5 *µ* m, 4.6 X 150mm manufactured by Nippon Waters) were used, and a mixed solution of 0.1% aqueous trifluoroacetic acid solution and 0.1%trifluoroacetic acid in acetonitrile was used as eluent. In other words, separation and purification were carried out over 30 to 40 minutes using the above-described columns at a flow rate of 1.5 mL/min while increasing over time the amount of the trifluoroacetic acid/acetonitrile solution contained in the eluent (in a concentration gradient set from 10% to 80% by volume). The peptide eluted from the reverse phase column was detected at a wavelength of 220 nm using ultraviolet ray detector (490E Detector manufactured by Waters) and shown on a recording chart as the peaks.

The molecular weight of each eluted peptide was determined with MALDI-TOF MS (Matrix-Assisted Laser Desorption Time of Flight Mass Spectrometry), using Voyager DE RP (trademark) manufactured by PerSeptive Biosystems. As a result, it was confirmed that the target peptide was synthesized and purified.

### < Example 2: Antimicrobial activity of synthetic peptides>

Regarding the antimicrobial peptides (samples 1 to 12) and the peptide of comparative sample 1 related to the present invention, their antimicrobial activities (minimum inhibitory concentration: MIC) against Gram-negative bacteria (Escherichia coli) and Gram-positive bacteria (Staphylococcus aureus) were determined, as described below, by liquid medium dilution method using a 96-well microplate.

First, a drug (synthesized peptide) solution at a concentration of 40 times the highest test concentration was prepared with sterile distilled water, and thereafter liquid broth media ("NUTRIENT BROTH Dehydrated" manufactured by DIFCO) at peptide concentrations of 200, 100, 50, 25, 12.5, 6.25, 3.13, and 1.56 µM were produced respectively. The prepared liquid broth media containing the peptide at each concentration described above were then loaded onto 96-well microplates in 100-µL aliquots.

At the same time, the tested bacteria cultured for 18 hours at 37°C on an agar plate ("Muller Hinton Agar" manufactured by DIFCO) were collected by scraping with a loop, and then suspended in sterilized physiological saline. 5µL of bacterial suspension prepared to be equivalent to 2 × 10⁷ cells/mL was inoculated (number of bacteria to be tested: approximately 1 × 10⁶cells/mL) into each well of the above-described microplate filled with the liquid broth media containing the peptide at each concentration. After inoculation, the microplate was then incubated in an incubator at 37°C, and the presence of bacteria was evaluated based on the turbidity after 24 hours. The minimum drug concentration (polypeptide concentration), at which no increase in bacterial turbidity was detected at the time of measurement, was defined as MIC (minimum inhibitory concentration) (unit: µM) in the present embodiment. The results are shown in Table 2.

**Table 2**

| Antimicrobial Activities (MIC: *µ* M) | | |
|---|---|---|
| Sample No. | S. aureus | E. coli |
| Sample 1 | 3.13 | 6.25 |
| Sample 2 | 6.25 | 25 |
| Sample 3 | 6.25 | 12.5 |
| Sample 4 | 1.56 | 6.25 |
| Sample 5 | 3.13 | 3.13 |
| Sample 6 | 50 | n.d. |
| Sample 7 | 50 | n.d. |
| Sample 8 | 50 | n.d. |
| Sample 9 | 12.5 | n.d. |
| Sample 10 | 12.5 | n.d. |
| Sample 11 | 12.5 | n.d. |
| Sample 12 | 25 | n.d. |
| | | |
| Comparative Sample 1 | > 200 | >200 |

| | | |
|---|---|---|
| n.d.: not tested | | |

As evident in the results shown in Table 2, all antimicrobial peptides having an antimicrobial associated sequence (samples 1 to 12) exhibited excellent antimicrobial activities. Samples 1 to 5 exhibited particularly high antimicrobial activities against Gram-positive bacteria (in this example, S. aureus).

On the other hand, no antimicrobial activity was detected in a comparative synthetic peptide (comparative sample 1).

### <Example 3: Preparation of granules>

After 50 mg of sample 1 peptide was mixed with 50 mg of crystallized cellulose and 400 mg of lactose, 1 mL of a mixed solution of ethanol and water was added and the mixture was kneaded. The kneaded product was granulated by a regular method, and thus a granular formulation of the antimicrobial polypeptide as the main component (a granulated antimicrobial agent) was obtained.

Specific examples of the present invention have been described above, but they are only illustrative and not limiting the scope of the claims. All changes and modifications in the specific examples illustrated above are intended to be embraced in the technologies disclosed in the appended claims.

### [Industrial Applicability]

As described above, since the antimicrobial peptides of the present invention possesses high antimicrobial activity, it can be utilized in medicines and hygiene goods.

## Claims

1. A pharmaceutical composition for use in preventing or treating a bacterial infection, the composition comprising:
a peptide having antimicrobial property against at least one kind of bacteria ; and
a pharmaceutically acceptable carrier;
wherein the peptide is composed of an amino acid sequence selected from the amino acid sequences below :
TLKERCLQVVRSLVK (SEQ ID NO: 1);
SLQYLCRFVIRQYTR (SEQ ID NO: 2);
SLQHICRMSIRRVMS (SEQ ID NO: 3);
TLLSLCRVAVRRALG (SEQ ID NO: 4);
TLKKRCLQVVRKLVK (SEQ ID NO: 5)
TLQHLCRKTVNGHLD (SEQ ID NO: 7);
SLQHICRTVICNCTT (SEQ ID NO: 8);
SLQYICRAVICRCTT (SEQ ID NO: 9);
SLQHLCRFRIRQLVR (SEQ ID NO: 10);
SLKHLCRKALRSFLT (SEQ ID NO: 11);
PLAHLCRLRVRKAIG (SEQ ID NO: 12); and
SLTHLCRLEIRSSIK (SEQ ID NO: 13), or
a modified amino acid sequence of the selected amino acid sequence in which one, two or three amino acid residues are conservatively substituted.

2. A peptide having antimicrobial property against at least one kind of bacteria, wherein the peptide consists of the amino acid sequence:
TLKKRCLQVVRKLVK (SEQ ID NO: 5).

3. A polynucleotide consisting of a nucleotide sequence encoding the peptide of claim 2, and/or a nucleotide sequence complementary to said nucleotide sequence.

4. A method for producing a peptide having antimicrobial property against at least one kind of bacteria, the method comprising:
designing a peptide composed of the amino acid sequence:
TLKKRCLQVVRKLVK (SEQ ID NO: 5),
and
synthesizing the designed peptide chain.

## Patentansprüche

1. Pharmazeutische Zusammensetzung zur Verwendung beim Vorbeugen oder Behandeln einer bakteriellen Infektion, wobei die Zusammensetzung umfasst:
ein Peptid mit einer antimikrobiellen Eigenschaft gegen mindestens eine Art von Bakterien; und
einen pharmazeutisch verträglichen Träger;
wobei das Peptid aus einer Aminosäuresequenz, die aus den nachfolgenden Aminosäuresequenzen ausgewählt ist, zusammengesetzt ist:
TLKERCLQVVRSLVK (SEQ ID NO: 1);
SLQYLCRFVIRQYTR (SEQ ID NO: 2);
SLQHICRMSIRRVMS (SEQ ID NO: 3);
TLLSLCRVAVRRALG (SEQ ID NO: 4);
TLKKRCLQVVRKLVK (SEQ ID NO: 5)
TLQHLCRKTVNGHLD (SEQ ID NO: 7);
SLQHICRTVICNCTT (SEQ ID NO: 8);
SLQYICRAVICRCTT (SEQ ID NO: 9);
SLQHLCRFRIRQLVR (SEQ ID NO: 10);
SLKHLCRKALRSFLT (SEQ ID NO: 11);
PLAHLCRLRVRKAIG (SEQ ID NO: 12); und
SLTHLCRLEIRSSIK (SEQ ID NO: 13), oder
eine modifizierte Aminosäuresequenz der ausgewählten Aminosäuresequenz, in der ein, zwei oder drei Aminosäurereste konservativ ausgetauscht sind.

2. Peptid mit einer antimikrobiellen Eigenschaft gegen mindestens eine Art von Bakterien, wobei das Peptid aus der Aminosäuresequenz:
TLKKRCLQVVRKLVK (SEQ ID NO: 5)
besteht.

3. Polynukleotid bestehend aus einer Nukleotidsequenz, die das Peptid nach Anspruch 2 kodiert, und/oder einer Nukleotidsequenz, die zu der Nukleotidsequenz komplementär ist.

4. Verfahren zum Herstellen eines Peptids mit einer antimikrobiellen Eigenschaft gegen mindestens eine Art von Bakterien, das Verfahren umfassend:
Entwerfen eines Peptids, das aus der Aminosäuresequenz:
TLKKRCLQVVRKLVK (SEQ ID NO: 5)
zusammengesetzt ist,
und
Synthetisieren der entworfenen Peptidkette.

## Revendications

1. Composition pharmaceutique pour utilisation dans la prévention ou le traitement d'une infection bactérienne, la composition comprenant :
un peptide possédant une propriété antimicrobienne contre au moins un type de bactérie ; et
un support pharmaceutiquement acceptable ;
dans laquelle le peptide est composé d'une séquence d'acides aminés choisie parmi les séquences d'acides aminés ci-dessous :
TLKERCLQVVRSLVK (SEQ ID NO: 1);
SLQYLCRFVIRQYTR (SEQ ID NO: 2);
SLQHICRMSIRRVMS (SEQ ID NO: 3);
TLLSLCRVAVRRALG SEQ ID NO: 4);
TLKKRCLQVVRKLVK (SEQ ID NO: 5) TLQHLCRKTVNGHLD (SEQ ID NO: 7);
SLQHICRTVICNCTT (SEQ ID NO: 8);
SLQYICRAVICRCTT (SEQ ID NO: 9);
SLQHLCRFRIRQLVR (SEQ ID NO: 10);
SLKHLCRKALRSFLT (SEQ ID NO: 11);
PLAHLCRLRVRKAIG (SEQ ID NO: 12); et SLTHLCRLEIRSSIK (SEQ ID NO: 13), ou
une séquence d'acides aminés modifiée de la séquence d'acides aminés sélectionnée dans laquelle un, deux ou trois résidus d'acide aminé sont substitués de manière conservative.

2. Peptide possédant une propriété antimicrobienne contre au moins un type de bactérie, dans lequel le peptide consiste en la séquence d'acides aminés :
TLKKRCLQVVRKLVK (SEQ ID NO: 5).

3. Polynucléotide consistant en une séquence nucléotidique codant le peptide selon la revendication 2, et/ou une séquence nucléotidique complémentaire à ladite séquence nucléotidique.

4. Procédé de production d'un peptide possédant une propriété antimicrobienne contre au moins un type de bactérie, le procédé comprenant :
la conception d'un peptide composé de la séquence d'acides aminés :
TLKKRCLQVVRKLVK (SEQ ID NO: 5),
et
la synthèse de la chaîne peptidique conçue.


### [Sequence List]

### sequence list .txt

### SEQUENCE LISTING

<110> TOAGOSEI CO., LTD
<120> ANTIMICROBIAL PEPTIDE AND USE THEREOF
<130> TG06-001PCT
<150> JP2005-037973 <151> 2005-02-15
<160> 13
<170> PatentIn version 3.1
<210> 1
   <211> 15
   <212> PRT
   <213> Artificial sequence
<220>
   <223> Designed antimicrobial peptide containing a terminal amide group
<400> 1
   Thr Leu Lys Glu Arg Cys Leu Gln Val Val Arg Ser Leu Val Lys
   1               5                   10                  15
<210> 2
   <211> 15
   <212> PRT
   <213> Artificial Sequence
<220>
   <223> Designed antimicrobial peptide containing a terminal amide group
<400> 2
   Ser Leu Gln Tyr Leu Cys Arg Phe Val Ile Arg Gln Tyr Thr Arg
   1               5                   10                  15
<210> 3
   <211> 15
   <212> PRT
   <213> Artificial Sequence
<220>
   <223> Designed antimicrobial peptide containing a terminal amide group
<400> 3
   Ser Leu Gln His Ile Cys Arg Met Ser Ile Arg Arg Val Met Ser
   1               5                   10                  15
<210> 4
   <211> 15
   <212> PRT
   <213> Artificial Sequence
<220>
   <223> Designed antimicrobial peptide containing a terminal amide group
<400> 4
   Thr Leu Leu Ser Leu Cys Arg Val Ala Val Arg Arg Ala Leu Gly
   1 5 10 15
<210> 5
   <211> 15
   <212> PRT
   <213> Artificial Sequence
<220>
   <223> Designed antimicrobial peptide containing a terminal amide group
<400> 5
   Thr Leu Lys Lys Arg Cys Leu Gln Val Val Arg Lys Leu Val Lys
   1               5                   10                  15
<210> 6
   <211> 15
   <212> PRT
   <213> Artificial Sequence
<220>
   <223> Designed peptide containing a terminal amide group
<400> 6
   Thr Leu His Gln Gln Cys Ile Arg Val Leu Lys Asn Asn Ile Asp
   1               5                   10                  15
<210> 7
   <211> 15
   <212> PRT
   <213> Artificial Sequence
<220>
   <223> Designed antimicrobial peptide containing a terminal amide group
<400> 7
   Thr Leu Gln His Leu Cys Arg Lys Thr Val Asn Gly His Leu Asp
   1               5                   10                  15
<210> 8
   <211> 15
   <212> PRT
   <213> Artificial Sequence
<220>
   <223> esigned antimicrobial peptide containing a terminal amide group
<400> 8
   Ser Leu Gln His Ile Cys Arg Thr Val Ile Cys Asn Cys Thr Thr
   1               5                   10                  15
<210> 9
   <211> 15
   <212> PRT
   <213> Artificial Sequence
<220>
   <223> Designed antimicrobial peptide containing a terminal amide group
<400> 9
   Ser Leu Gln Tyr Ile Cys Arg Ala Val Ile Cys Arg Cys Thr Thr
   1 5 10 15
<210> 10
   <211> 15
   <212> PRT
   <213> Artificial Sequence
<220>
   <223> Designed antimicrobial peptide containing a terminal amide group
<400> 10
   Ser Leu Gln His Leu Cys Arg Phe Arg Ile Arg Gln Leu Val Arg
   1 5 10 15
<210> 11
   <211> 15
   <212> PRT
   <213> Artificial Sequence
<220>
   <223> Designed antimicrobial peptide containing a terminal amide group
<400> 11
   Ser Leu Lys His Leu Cys Arg Lys Ala Leu Arg Ser Phe Leu Thr
   1 5 10 15
<210> 12
   <211> 15
   <212> PRT
   <213> Artificial Sequence
<220>
   <223> Designed antimicrobial peptide containing a terminal amide group
<400> 12
   Pro Leu Ala His Leu Cys Arg Leu Arg Val Arg Lys Ala Ile Gly
   1 5 10 15
<210> 13
   <211> 15
   <212> PRT
   <213> Artificial Sequence
<220>
   <223> Designed antimicrobial peptide containing a terminal amide group
<400> 13
   Ser Leu Thr His Leu Cys Arg Leu Glu Ile Arg Ser Ser Ile Lys
   1               5                   10                  15